(19) Europäisches Patentamt / European Patent Office / Office européen des brevets

(11) **EP 4 740 843 A1**

(12) **EUROPEAN PATENT APPLICATION**

(43) Date of publication:
**13.05.2026 Bulletin 2026/20**

(21) Application number: **25203543.1**

(22) Date of filing: **22.09.2025**

(51) International Patent Classification (IPC):
**A61B 5/00** *(2006.01)* **A61B 5/0507** *(2021.01)*

(52) Cooperative Patent Classification (CPC):
**A61B 5/0507; A61B 5/443; A61B 5/445; A61B 5/7264; A61B 5/7267; G16H 40/63; G16H 50/20; G16H 50/30; G16H 50/70**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **07.11.2024 IN 202421085558**

(71) Applicant: **Tata Consultancy Services Limited
Maharashtra (IN)**

(72) Inventors:
• **KHASNOBISH, Anwesha
700135 Kolkata, West Bengal (IN)**

• **MAZUMDER, Annesha
700135 Kolkata, West Bengal (IN)**
• **BHARADWAJ, Vedula Kiran
700156 Kolkata, West Bengal (IN)**
• **CHAKRAVARTY, Tapas
700135 Kolkata, West Bengal (IN)**
• **AKHTAR, Mohammad Jaleel
208016 Kanpur, Uttar Pradesh (IN)**

(74) Representative: **Goddar, Heinz J.
Boehmert & Boehmert
Anwaltspartnerschaft mbB
Pettenkoferstrasse 22
80336 München (DE)**

(54) **SYSTEM AND METHOD FOR UNOBTRUSIVE OEDEMA SEVERITY CLASSIFICATION AND QUANTIFICATION USING MICROWAVE SENSING**

(57) Monitoring and detection of oedema severity is crucial for effective treatment of the underlying ailments. Conventional methods involve setups that require harmful radiation, are expensive, tedious, and bulky. The present disclosure provides a system and method for unobtrusive oedema severity classification and quantification using microwave sensing. A vector network analyzer, a microstrip RF patch antenna customized for operation at 4 GHz under dielectric loading of a numerical human phantom and associated RF components are used for oedema severity monitoring of a sample under test. For oedema severity classification, a machine learning (ML) based approach is implemented making use of reflection parameters, and accordingly physics-based electromagnetic features are extracted. The physics-based electromagnetic features are used to classify the sample under test into a specific oedema severity category. Further, a water percentage is quantified using regression models for the sample under test based on the oedema severity category.

Acquiring, a plurality of scan measurements at a plurality of locations of a sample under test using a tuned antenna, wherein the tuned antenna is a microstrip patch antenna operating at a specific operating frequency with one or more optimized dimensions and positioned at a predefined distance from a numerical biological phantom — 202

Determining, a plurality of reflection parameters from the plurality of scan measurements at each location from the plurality of locations of the sample under test using a vector network analyzer, wherein the vector network analyzer utilizes microwave sensing for determining the plurality of reflection parameters — 204

Extracting, a plurality of physics-based electromagnetic (EM) features using a predetermined resonant frequency and a corresponding value of the plurality of reflection parameter at the predetermined resonant frequency to obtain a feature vector, wherein the feature vector comprises a training feature vector and a testing feature vector — 206

Classifying, via the one or more hardware processors, the sample under test into one of (i) a first oedema severity category and (ii) a second oedema severity category, using a trained classifier model for the testing feature vector, wherein the trained classifier model is trained using the training feature vector — 208

Quantifying, via the one or more hardware processors, the oedema based on a water percentage in the sample under test that is classified into the first oedema severity category and the second oedema severity category, using a trained regression model for a specific set of testing feature vector from the testing feature vector — 210

200

**FIG. 3**

EP 4 740 843 A1

**Description**

CROSS-REFERENCE TO RELATED APPLICATIONS AND PRIORITY

The present application claims priority to Indian application no. 202421085558, filed on November 07, 2024.

TECHNICAL FIELD

The disclosure herein generally relates to microwave sensing, and, more particularly, to a system and method for unobtrusive oedema severity classification and quantification using microwave sensing.

BACKGROUND

Oedema is accumulation of water in interstitial spaces beneath skin. It is not a disease in itself, rather occurs due to various underlying conditions including heart, kidney, liver diseases, certain medications and many more. Oedema monitoring and intervention thus becomes essential to effectively treat the underlying condition(s). However, typically oedema is only detected when water accumulation increases to such a great extent that swelling (i.e., physical deformation) occurs in peripheral areas (e.g., lower hands and/or legs). Hence, quantitative monitoring of oedema severity even before its physical manifestation becomes crucial.

Most frequently used method of clinical assessment of oedema is either visual inspection or pitting oedema scale. However, both these methods are prone to subjective assessment error. Other than these methods, imaging techniques like Lymphoscintigraphy, ultrasonography, and magnetic resonance imaging (MRI) can evaluate oedema, but they are not portable, use harmful radiation, and are costly with long preparation time. It is observed microwave based method has lot of potential for oedema severity detection. This makes it possible to monitor variations in tissue hydration levels, such as those associated with oedema. Advantages of using microwave technology include its non-invasive nature, potential for continuous monitoring, and ability to provide information about tissue properties at different depths. However, with microwave-based assessment of oedema, it becomes challenging to gather information about the tissue properties since air-skin interface reflects most of incident microwave energy.

Current microwave-based oedema or tissue hydration detection methods mostly rely on open-ended coaxial probe measurements, which are contact-based in nature and are incapable of assessing the extent of oedema typically beyond 2-3 mm depth. Other existing methods that have explored oedema assessment utilize either imaging-based approaches or contact-based sensors, which are unsuitable for efficient long-term monitoring. Microwave-based biomedical sensing suffers from a few critical challenges like antenna impedance mismatch due to proximity to biological medium, sensing resolution and penetration depth. For greater penetration and improved spatial resolution, the antenna should be placed near the biological media which in-turn affects a shift in resonance frequency of the antenna. Considering these impediments, it appears that conventional modalities for oedema detection either suffer from subjective assessment errors or involve setups that require harmful radiation, are expensive, tedious, and bulky.

SUMMARY

Embodiments of the present disclosure present technological improvements as solutions to one or more of the above-mentioned technical problems recognized by the inventors in conventional systems. For example, in one aspect, a processor implemented method is provided. The processor implemented method comprising acquiring, via one or more hardware processors, a plurality of scan measurements at a plurality of locations of a sample under test using a tuned antenna, wherein the tuned antenna is a microstrip patch antenna operating at a specific operating frequency with one or more optimized dimensions and positioned at a predefined distance from a numerical biological phantom; determining, via the one or more hardware processors, a plurality of reflection parameters from the plurality of scan measurements at each location from the plurality of locations of the sample under test using a vector network analyzer, wherein the vector network analyzer utilizes microwave sensing for determining the plurality of reflection parameters; extracting, via the one or more hardware processors, a plurality of physics-based electromagnetic (EM) features using a predetermined resonant frequency and a corresponding value of the plurality of reflection parameters at the predetermined resonant frequency to obtain a feature vector, wherein the feature vector comprises a training feature vector and a testing feature vector; classifying, via the one or more hardware processors, the sample under test into one of (i) a first oedema severity category, and (ii) a second oedema severity category, using a trained classifier model for the testing feature vector, wherein the trained classifier model is trained using the training feature vector; and quantifying, via the one or more hardware processors, the oedema based on a water percentage in the sample under test that is classified into the first oedema severity category and the second oedema severity category, using a trained regression model for a specific set of testing feature vector from the testing feature vector.

In another aspect, there is provided a system. The system includes a memory storing instructions; one or more communication interfaces; and one or more hardware processors coupled to the memory via the one or more communication interfaces, wherein the one or more hardware processors are configured by the instructions to: acquire a plurality of scan measurements at a plurality of locations of a sample under test using a tuned antenna, wherein the tuned antenna is a microstrip patch antenna operating at a specific operating frequency with one or more optimized dimensions and positioned at a predefined distance from a numerical biological phantom; determine a plurality of reflection parameters from the plurality of scan measurements at each location from the plurality of locations of the sample under test using a vector network analyzer, wherein the vector network analyzer utilizes microwave sensing for determining the plurality of reflection parameters; extract a plurality of physics-based electromagnetic (EM) features using a predetermined resonant frequency and a corresponding value of the plurality of reflection parameter at the predetermined resonant frequency to obtain a feature vector, wherein the feature vector comprises a training feature vector and a testing feature vector; classify the sample under test into one of (i) a first oedema severity category and (ii) a second oedema severity category, using a trained classifier model for the testing feature vector, wherein the trained classifier model is trained using the training feature vector; and quantify the oedema based on a water percentage in the sample under test that is classified into the first oedema severity category and the second oedema severity category, using a trained regression model for a specific set of testing feature vector from the testing feature vector.

In yet another aspect, there are provided one or more non-transitory machine readable information storage mediums comprising one or more instructions which when executed by one or more hardware processors causes at least one of: acquiring a plurality of scan measurements at a plurality of locations of a sample under test using a tuned antenna, wherein the tuned antenna is a microstrip patch antenna operating at a specific operating frequency with one or more optimized dimensions and positioned at a predefined distance from a numerical biological phantom; determining a plurality of reflection parameters from the plurality of scan measurements at each location from the plurality of locations of the sample under test using a vector network analyzer, wherein the vector network analyzer utilizes microwave sensing for determining the plurality of reflection parameters; extracting a plurality of physics-based electromagnetic (EM) features using a predetermined resonant frequency and a corresponding value of the plurality of reflection parameters at the predetermined resonant frequency to obtain a feature vector, wherein the feature vector comprises a training feature vector and a testing feature vector; classifying the sample under test into one of (i) a first oedema severity category, and (ii) a second oedema severity category, using a trained classifier model for the testing feature vector, wherein the trained classifier model is trained using the training feature vector; and quantifying the oedema based on a water percentage in the sample under test that is classified into the first oedema severity category and the second oedema severity category, using a trained regression model for a specific set of testing feature vector from the testing feature vector.

In accordance with an embodiment of the present disclosure, the sample under test is a biological tissue.

In accordance with an embodiment of the present disclosure, the specific operating frequency of the tuned antenna is 4 Giga Hertz (GHz) and the predefined distance of the tuned antenna from the numerical human phantom is 10 millimeter (mm).

In accordance with an embodiment of the present disclosure, the plurality of physics-based electromagnetic (EM) features specify water accumulation in the sample under test that represents a detected oedema.

In accordance with an embodiment of the present disclosure, the first oedema severity category represents a low oedema severity category, and the second oedema severity category represents a medium oedema severity category.

In accordance with an embodiment of the present disclosure, the step of classifying comprises classifying the sample under test into a third oedema severity category that represents a low oedema severity category.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates a system for unobtrusive oedema severity classification and quantification using microwave sensing, according to some embodiments of the present disclosure.

FIG. 2 is an exemplary block diagram of a system of FIG. 1 implementing a tuned antenna for unobtrusive oedema severity classification and quantification using microwave sensing, according to some embodiments of the present disclosure.

FIG. 3 is an exemplary flow diagram illustrating method for unobtrusive oedema severity classification and quantification using microwave sensing, according to some embodiments of the present disclosure.

FIGS. 4A and 4B provides a diagram illustrating placement of a conventional untuned antenna and tuned antenna respectively for unobtrusive oedema severity classification and quantification using microwave sensing, according to some embodiments of the present disclosure.

FIGS. 5A and 5B depict graphical representations of simulation frequency response of the conventional untuned antenna and tuned antenna respectively for unobtrusive oedema severity classification and quantification using microwave sensing, according to some embodiments of the present disclosure.

FIGS. 6A through 6D depict diagram representations illustrating an experimental setup and preparation for unobtrusive oedema severity classification and quantification using microwave sensing, according to some embodiments of the present disclosure.

FIGS. 7A through 7C depict diagram representations illustrating different experimental scenarios for unobtrusive oedema severity classification and quantification using microwave sensing, according to some embodiments of the present disclosure.

FIGS. 8A through 8C depict exemplary graphical representations illustrating trends of each of the plurality of EM features for different experimental scenarios for untuned antennas and tuned antennas, according to an embodiment of the present disclosure.

FIG. 9 illustrates a confusion matrix for a classifier used for unobtrusive oedema severity classification and quantification using microwave sensing, according to an embodiment of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Monitoring and detection of oedema severity is crucial for effective treatment of the underlying ailments. It is observed that microwave based method has lot of potential for oedema severity detection. This makes it possible to monitor variations in tissue hydration levels, such as those associated with oedema. Microwave imaging for biomedical applications quite often uses non-contacting scheme with the help of radio frequency (RF) antennas, where measured reflected or transmitted microwave signals are analyzed to extract information about a tissue dielectric property. Measured reflection data is dependent upon complex permittivity of the tissues, which can be correlated with their composition. However, conventional microwave imaging based modalities for oedema detection either suffer from subjective assessment errors or involve setups that require harmful radiation, are expensive, tedious, and bulky.

There is a need for an approach to address the problem of convention methods using microwave biomedical sensing for detecting severity of oedema. Embodiments of the present disclosure provide a system and method for unobtrusive oedema severity classification and quantification using microwave sensing. The present disclosure provides an unobtrusive microwave-based sensing methodology utilizing a vector network analyzer, a customized tuned antenna and associated RF components for oedema severity monitoring. A microstrip RF patch antenna is customized for operation at a specific frequency (e.g., 4 GHz) under dielectric loading of a numerical human phantom. The performance of the customized tuned antenna is compared with that of a corresponding standard patch antenna at the specific frequency (e.g., 4 GHz) to emphasize its significance for oedema detection. The method of the present disclosure is validated by establishing an experimental setup emulating realistic oedema scenarios, and a diverse dataset is built by measuring a reflection data at the designated frequency under various conditions. For oedema severity classification, a machine learning (ML) based approach is implemented making use of the extensive reflection data, and accordingly physics-based features are extracted. Subsequently, a ML assisted oedema severity (Low, Medium and High) classification, is carried out with a remarkable accuracy of $93.78 \pm 4.15\%$.

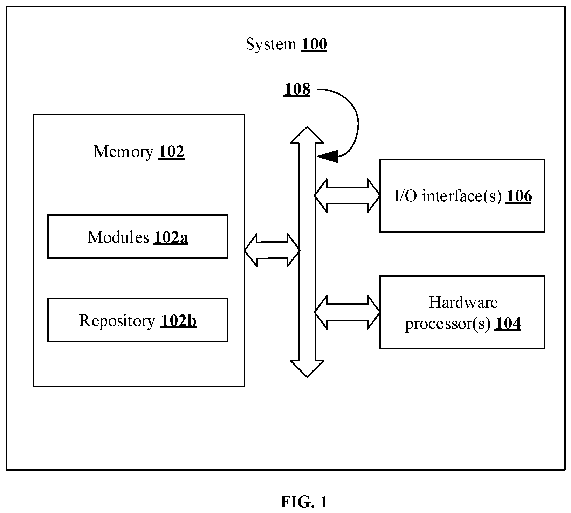

Referring now to the drawings, and more particularly to FIGS. 1 through 9, where similar reference characters denote corresponding features consistently throughout the figures, there are shown preferred embodiments and these embodiments are described in the context of the following exemplary system and/or method.

FIG. 1 illustrates a system 100 for unobtrusive oedema severity classification and quantification using microwave sensing, according to some embodiments of the present disclosure, according to an embodiment of the present disclosure. In an embodiment, the system 100 includes or is otherwise in communication with one or more hardware processors 104, communication interface device(s) or input/output (I/O) interface(s) 106, and one or more data storage devices or memory 102 operatively coupled to the one or more hardware processors 104. The one or more hardware processors 104, the memory 102, and the I/O interface(s) 106 may be coupled to a system bus 108 or a similar mechanism. The system 100 is further connected to a vector network analyzer and an antenna unit (not shown in FIG. 1) via the I/O interface(s) 106.

The I/O interface(s) 106 may include a variety of software and hardware interfaces, for example, a web interface, a graphical user interface, and the like. The I/O interface(s) 106 may include a variety of software and hardware interfaces, for example, interfaces for peripheral device(s), such as a keyboard, a mouse, an external memory, a plurality of sensor devices, a printer and the like. Further, the I/O interface(s) 106 may enable the system 100 to communicate with other devices, such as web servers and external databases.

The I/O interface(s) 106 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface(s) 106 may include one or more ports for connecting a number of computing systems with one another or to another server computer. Further, the I/O interface(s) 106 may include one or more ports for connecting a number of devices to one another or to another server.

The one or more hardware processors 104 may be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In the context of the present disclosure, the expressions 'processors' and 'hardware processors' may be used interchangeably. In an embodiment, the system 100 can be implemented in a variety of computing systems, such as laptop computers, portable computer, notebooks, hand-held devices, workstations, mainframe computers, servers, a network cloud and the like.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 102 includes a plurality of modules 102a and a repository 102b for storing data processed, received, and generated by one or more of the plurality of modules 102a. The plurality of modules 102a may include routines, programs, objects, components, data structures, and so on, which perform particular tasks or implement particular abstract data types.

The plurality of modules 102a may include programs or computer-readable instructions or coded instructions that supplement applications or functions performed by the system 100. The plurality of modules 102a may also be used as, signal processor(s), state machine(s), logic circuitries, and/or any other device or component that manipulates signals based on operational instructions. Further, the plurality of modules 102a can be used by hardware, by computer-readable instructions executed by the one or more hardware processors 104, or by a combination thereof. Further, the memory 102 may include information pertaining to input(s)/output(s) of each step performed by the processor(s) 104 of the system 100 and methods of the present disclosure.

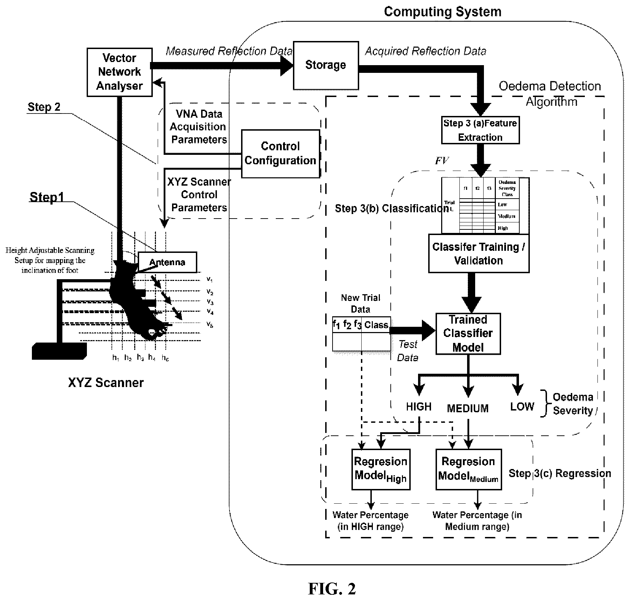

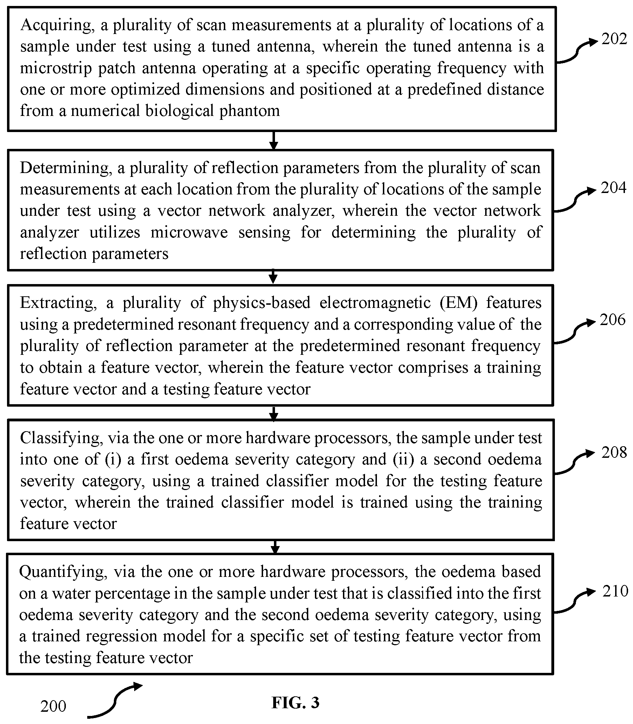

The repository 102b may include a database. Further, the repository 102b amongst other things, may serve as a database or includes a plurality of databases for storing the data that is processed, received, or generated as a result of the execution of the plurality of modules 102a. Although the repository 102b is shown internal to the system 100, it will be noted that, in alternate embodiments, the repository 102b can also be implemented external to the system 100, where the repository 102b may be stored within an external database (not shown in FIG. 1) that is communicatively coupled to the system 100. The data contained within such external database may be periodically updated. For example, new data may be added into the external database and/or existing data may be modified and/or non-useful data may be deleted from the external database. In one example, the data may be stored in an external system, such as a Lightweight Directory Access Protocol (LDAP) directory and a Relational Database Management System (RDBMS). In another embodiment, the data stored in the repository 102b may be distributed between the system 100 and the external database. Functions of the components of the system 100 are now explained with reference to architecture as depicted in steps in flow diagrams in FIG. 2 and FIG. 3.

FIG. 2 is an exemplary block diagram of the system of FIG. 1 implementing a tuned antenna for unobtrusive oedema severity classification and quantification using microwave sensing, according to some embodiments of the present disclosure. The system 100 includes at least one of (i) a microstrip conventional patch antenna 202A (i.e., alternatively referred as an untuned antenna (UTA)) placed in a free space, and (ii) a tuned antenna 202B, a sample under test 204 kept at a distance from the tuned antenna, and a vector network analyzer 206 (i.e., mini circuits eVNA63+) working in a range of 300 KHz-6 GHz, connected through a SMA cable to at least one of (i) the tuned antenna and (ii) the untuned antenna. In an embodiment, the untuned antenna is operating at specific frequency (e.g., 4 GHz) and designed under CST Microwave Studio environment on a 1.6 mm thick RT Duroid 5880 substrate.

FIG. 3, with reference to FIGS. 1 and 2, is an exemplary flow diagram illustrating method for unobtrusive oedema severity classification and quantification using microwave sensing, according to some embodiments of the present disclosure., according to an embodiment of the present disclosure. Referring to FIG. 3, in an embodiment, the system(s) 100 comprises one or more data storage devices or the memory 102 operatively coupled to the one or more hardware processors 104 and is configured to store instructions for execution of steps of the method by the one or more processors 104. The steps of the method 200 of the present disclosure will now be explained with reference to components of the system 100 of FIG. 1, the block diagram of FIG. 2, the flow diagram as depicted in FIG. 3, and one or more examples. Although steps of the method 200 including process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any practical order. Further, some steps may be performed simultaneously, or some steps may be performed alone or independently.

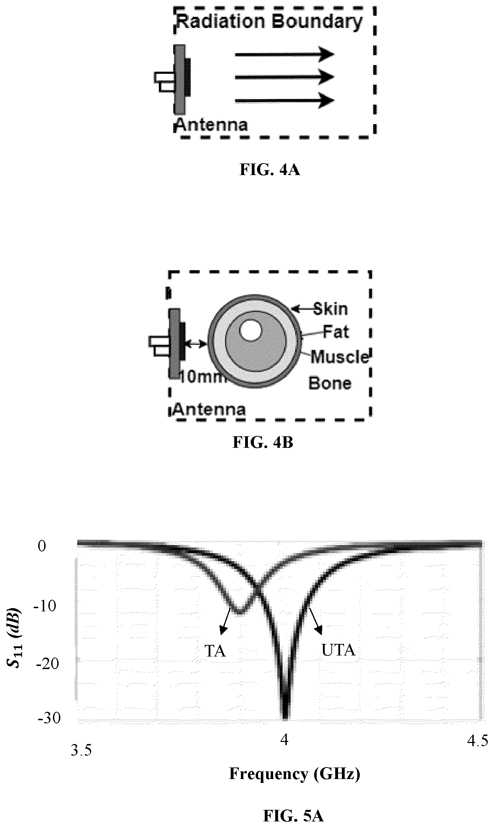

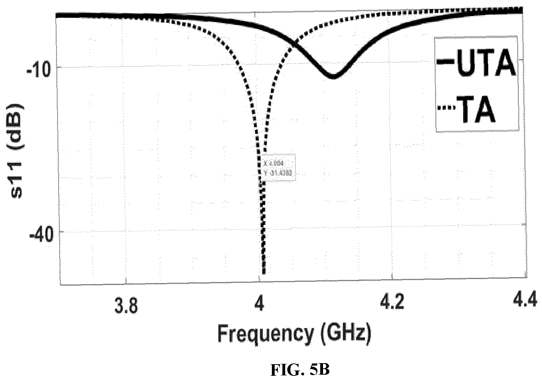

In an embodiment, at step 302 of the present disclosure, the one or more hardware processors 104 are configured to acquire a plurality of scan measurements at a plurality of locations of a sample under test using a tuned antenna. A 3D (XYZ) scanner is used to acquire the plurality of scan measurements of sample under test. In an embodiment, the sample under test is a biological tissue. In the present disclosure, a human feet is considered as the sample under test. Position of 3D (XYZ) scanner is controlled via a computer. For a particular spacing between the antenna and the sample under test denoted by 'z', the 3D scanner stops at specific positions $(x_i, y_i)$, for specific time and then moves to the next $(x_i, y_i)$ position, where ($i$ = 1, 2 ... $P$). The tuned antenna is a microstrip patch antenna operating at a specific operating frequency with one or more optimized dimensions and positioned at a predefined distance from a numerical biological phantom. The specific operating frequency of the tuned antenna is 4 Giga Hertz (GHz). The predefined distance of the tuned antenna from the numerical human phantom is 10 millimeter (mm). FIGS. 4A and 4B provides a diagram illustrating placement of the conventional untuned antenna and tuned antenna respectively for unobtrusive oedema severity classification and quantification using microwave sensing, according to some embodiments of the present disclosure. FIGS. 5A and 5B depict graphical representations of simulation frequency response of the conventional untuned antenna and tuned antenna respectively for unobtrusive oedema severity classification and quantification using microwave sensing, according to some embodiments of the present disclosure. A simulated gain and 3 dB beamwidth of the untuned antenna are found to be 7.83 dBi and 78.30°, respectively. Operating frequency of 4 GHz is a trade-off between compact size, depth of penetration, a better spatial resolution, and for a lower loss inside skin. In the present disclosure, a customized patch antenna operating at 4 GHz is designed by placing the numerical biological phantom (i.e., human phantom) 10 mm away from the tuned antenna as shown in FIGS. 4B, 5A and 5B to make it more suitable for biomedical application. The tuned antenna is a customized antenna for biomedical application since presence of a highly lossy dielectric in proximity to the untuned antenna results into a shift in its resonance frequency and would also cause impedance mismatch. It appears that this issue can be mitigated by altering the microstrip conventional patch antenna dimensions such as length, width and feed probe position to obtain better impedance match at a desired resonance frequency. It is observed that the performance of this tuned antenna may sometime be dependent on relative position, angle and electrical properties of the interrogating medium. Table 1 gives dimensions of the untuned antenna (i.e., conventional microstrip patch antenna) and the tuned antenna.

Table 1

| Parameters | Resonant Frequency | Length | Width |
|---|---|---|---|
| **Untuned Antenna (UTA)** | 4GHz | 29.5mm | 23.4mm |
| **Tuned Antenna (TA)** | 4GHz | 32mm | 24mm |

At step 304 of the present disclosure, the one or more hardware processors 104 are configured to determine a plurality of reflection parameters from the plurality of scan measurements at each location from the plurality of locations of the sample under test using a vector network analyzer. The vector network analyzer utilizes microwave sensing for determining the plurality of reflection parameters. A sample under test is kept at 10 mm from the tuned antenna for all cases and a frequency vs reflection parameter which is $S_{11}$(dB) are recorded for each case. At every specific $(x_i, y_i)$ position, the vector network analyzer is controlled with computer to transmit a microwave and then receive the reflected data (reflection parameter, $S_{11}$ data).

Further, at step 306 of the present disclosure, the one or more hardware processors 104 are configured to extract a plurality of physics-based electromagnetic (EM) features using a predetermined resonant frequency and a corresponding value of the plurality of reflection parameters at the predetermined resonant frequency to obtain a feature vector. The feature vector comprises a training feature vector and a testing feature vector. From the plurality of reflection parameters (Alternatively referred as recorded reflection) $S_{11}$ data, three distinctive physics-based electromagnetic (EM) features are computed. For this purpose, two parameters namely $f_{res}$ and $S_{11}$ (res) representing a resonance frequency and a value of reflection parameter ($S_{11}$) at the resonance frequency, respectively are considered. A third parameter termed as fractional bandwidth (FracBW) is computed using equation (1) below:

$$FracBW = \frac{f_U - f_L}{f_{res}} \tag{1}$$

Here, $f_U$ and $f_L$ represent frequency points corresponding to $S_{11}$ = -10 dB. For all the three parameters, a percentage change is calculated for each oedema case with respect to a healthy/no-water case as shown in equations (2) to (5). These

three parameters, *FracBW(%)*, $\Delta f_{res}(\%)$, $S_{11}(res)(\%)$, and $S_{11}(f_{res\text{-}healthy})(\%)$ are used as the plurality of EM features $f_1$, $f_2$, $f_3$, and $f_4$ respectively.

$$f_1: FracBW(\%) = \left[\frac{\text{FracBW (Oedema)} - \text{FracBW(Healthy)}}{FracBW\ (Healthy)}\right] \times 100 \quad (2)$$

$$f_2: \Delta f_{res}(\%) = \left[\frac{f_{res}\ (\text{Oedema}) - f_{res}\ (Healthy)}{f_{res}\ (Healthy)}\right] \times 100 \quad (3)$$

$$f_3: S_{11}(res)(\%) = \left[\frac{S_{11}(res)|(Oedema) - S_{11}(res)|(Healthy)}{S_{11}\ (res)|(Healthy)}\right] \times 100 \quad (4)$$

$$f_4: S_{11}\big(f_{res-healthy}\big)(\%) =$$

$$\left[\frac{S_{11}\big(f_{res-healthy}\big)|(Oedema) - S_{11}\big(f_{res-healthy}\big)|(Healthy)}{S_{11}\big(f_{res-healthy}\big)|(Healthy)}\right] \times 100 \quad (5)$$

In an embodiment, the plurality of physics-based electromagnetic (EM) features specify water accumulation in the sample under test that represents a detected oedema The above four features extracted from various positions ($x_i$, $y_i$) of various scan measurements form the feature vector (*FV*) which is used to train and validate a classifier model. The feature vector is split into a training feature vector and a testing feature vector. The training feature vector is represented by $FV_{Train}$ which is a $M \times N$ matrix, where $M$ is the number of trials (i.e., repetitions of data acquisition), and $N = 4$ which is obtained from a product of numbers of features and total number of positions that the 3D scanner stopped for acquiring data as ({*f1, f2, f3, f4*}) $\times P$, where $P$ represents total number of positions that the 3D scanner stopped for acquiring data.

At step 308 of the present disclosure, the one or more hardware processors 104 are configured to classify the sample under test into one of (i) a first oedema severity category, and (ii) a second oedema severity category, using a trained classifier model for the testing feature vector. The trained classifier model is trained using the training feature vector. The testing feature vector from remaining trials (which are not included in training set $FV_{Train}$) and represented by $FV_{Test}$ is then used to test the classifier performance. The plurality of physics-based electromagnetic (EM) features are classified using support vector machine classifier with quadratic kernel (degree 2) and referred as $SVM_{Quad}$, which implicitly maps input features into a higher-dimensional space. In this transformed space, the SVM constructs a hyperplane that translates into a quadratic boundary in original feature space. This capability makes quadratic SVMs particularly effective for datasets used in the present disclosure, where classes are not linearly separable and for multiclass classification problems, providing more flexibility and improved accuracy in modeling intricate patterns within the data. In an embodiment, the first oedema severity category represents a high oedema severity category and the second oedema severity category represents a medium oedema severity category. In an embodiment, the step of classifying comprises classifying the sample under test into a third oedema severity category that represents a low oedema severity category. For the oedema severity categorization, percentage of water accumulation <5% is termed as low, 5-20% is termed as medium and >20% is termed as high oedema severity category. The SVM classifier is trained and validated where the input is $FV_{Train}$ and a trained classifier model (*Classifier$_{Trained}$*) is obtained at the output. Then $FV_{Test}$ is inputted to *Classifier$_{Trained}$* whose output is one of the oedema severity class: {Low, Medium, High}. If the output is low, then the processing stops there.

Further, at step 310 of the present disclosure, the one or more hardware processors 104 are configured to quantify the oedema based on a water percentage in the sample under test that is classified into the first oedema severity category and the second oedema severity category, using a trained regression model for a specific set of testing feature vector from the testing feature vector. The specific set of testing feature vector is a subset of the testing feature vector which has not been classified into low oedema severity category and represented by $FV'_{Test}$. Two regression models represented by *Regression Model$_{medium}$* and *Regression Model$_{high}$* are modelled using inputs $FV_{RegTrainMedium}$ and $FV_{RegTrainHigh}$ respectively containing respective training feature vectors. Now the $FV'_{Test}$ is made input to either *Regression Model$_{medium}$* or *Regression Model$_{high}$* depending upon the output {Medium} or {High} of the step 308 and the corresponding output is an approximate *Water Percentage*.

**Experimental Setup:**

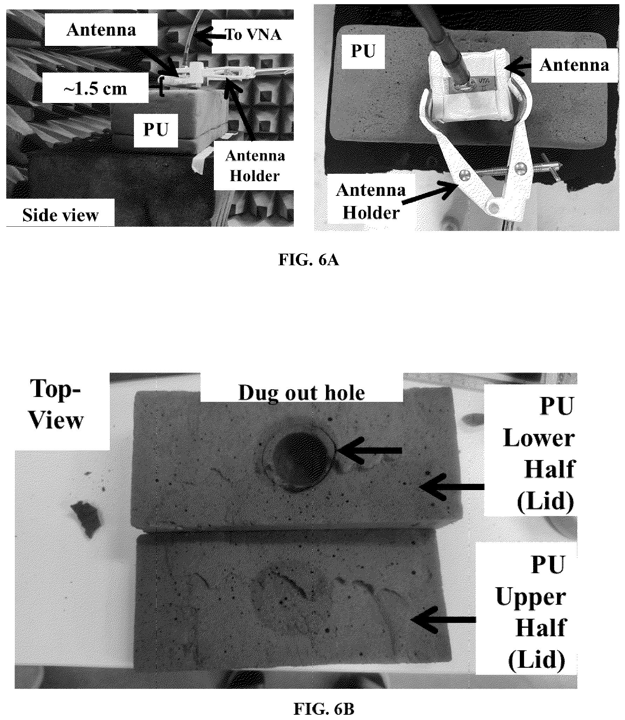

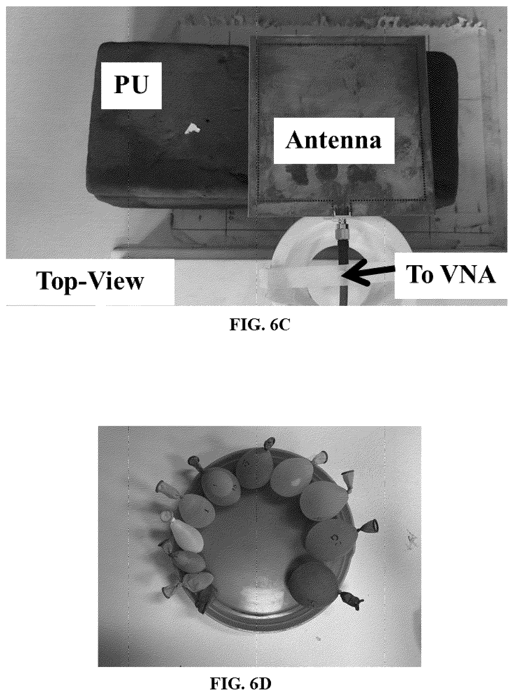

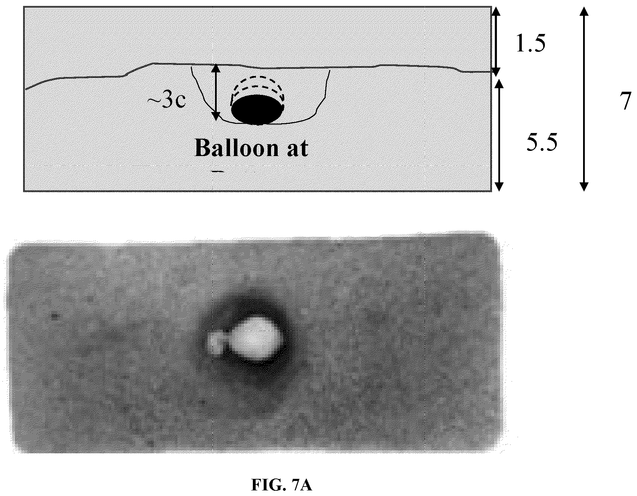

In the present disclosure, experiments have been conducted to ascertain feasibility of non-contact detection of increased water volume percentage using reflected microwave signals. FIGS. 6A through 6D depict diagram representations illustrating an experimental setup and preparation for unobtrusive oedema severity classification and quantification using microwave sensing, according to some embodiments of the present disclosure. FIGS. 7A through 7C depict diagram representations illustrating different experimental scenarios for unobtrusive oedema severity classification and quantification using microwave sensing, according to some embodiments of the present disclosure. For conducting experiments, a 22 cm $\times$ 9.5 cm $\times$ 7 cm block of polyurethane (PU) foam, as shown in FIGS. 6A through 7C, was taken as the numerical biological phantom due to its porosity, inhomogeneity, intrinsic water retention capability (synonymous to biological tissues) and its configurable nature. Further, dielectric properties of PU foam could be easily altered by changing only the water content inside it. It was noted that the tuned antenna is primarily designed for the human phantom, and hence under actual measurement scenario the results would be more accurate for increased water volume, when a PU foam is used as a bare human phantom. Measurement procedure involved recording an effect of varying content of water on antenna reflection characteristics, and also a comparison of the detection sensitivity of the two antennas (i.e., UTA and TA).

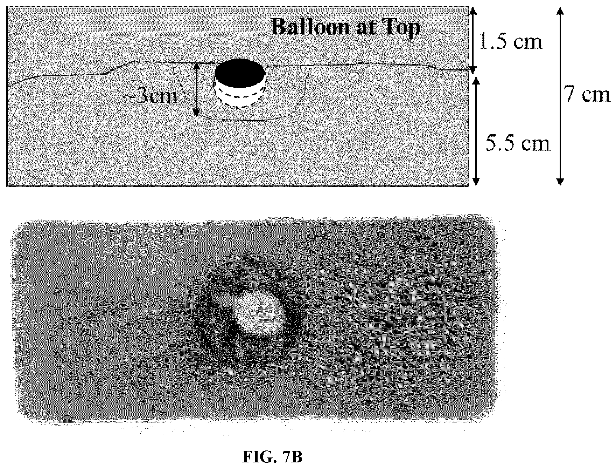

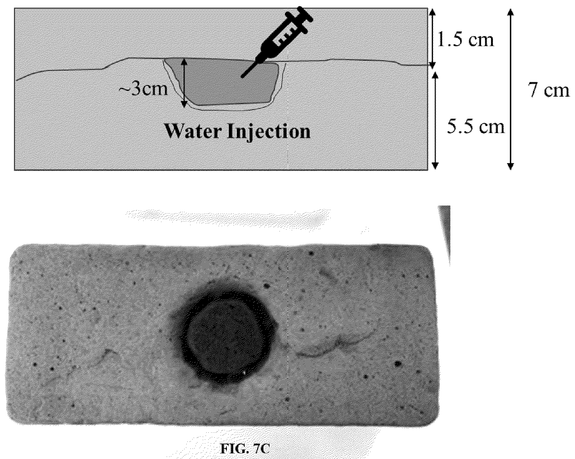

Oedema is primarily related to water retention in interstitial spaces in tissues underneath/on skin. However, there is no fixed path or channel of this water accumulation. This is due to an inherent inhomogeneity of tissue structures and absence of any point source of water accumulation. Keeping these in mind, experiments were conducted in three parts (Exp-1, Exp-2 and Exp-3) so as to emulate oedema in various realistic possibilities as shown in FIGS. 7A through 7C. In all the experiments, water was kept constrained in a definite area so as to observe a change in reflection due to change in water accumulation only. The PU foam was cut into two halves, where a lid foam (i.e., upper half) was of height 1.5 cm. From lower half of the PU foam, a hole was scooped out with a depth of about 3 cm, and a diameter of approximately 4 cm as shown in FIG. 6B. As shown in FIGS. 7A and 7B, Exp-1 and Exp-2 deals with water inserted in balloons, and placed subsequently inside this hole in the PU foam. Further, as shown in FIG. 7C, in Exp-3, water was injected in a piece of foam inserted in the hole. These experiments were designed in a manner so as to vary only one parameter physically, i.e., water content, and its effect was assessed on reflected microwaves. Specifically, in Exp-1, the balloon was kept in hole such that it was at the bottom. As the amount of water in the balloon increases, the balloon grows upwards as well as sidewards. Ultimately, largest water filled balloon with 30ml fills dug-out hole completely. In Exp-2, the dugout hole was first filled with PU foam filler pieces and the balloon was kept at the top of the filler material, thus ensuring that there was no empty space inside the hole. As the volume of the waterfilled balloon increases, it extends downwards and amount of cushioning filler material drops. Again, for case of maximum water volume, the dug-out hole was filled completely by the balloon only. Thus, while in Exp-1, distance from antenna to nearest surface of water (in balloon) decreases with increasing water volume, the distance remained constant in Exp-2. Finally, in Exp-3, a PU foamcutout with varying levels of injected water was used to fill in the dugout hole. This was done to model the uncertainty of water accumulation and distribution, while keeping it constrained to the specified volume. Multiple trials for each of the three experiments have been conducted with both the antenna.

**Experimental Results**

In an embodiment, experimental results have been obtained to validate effectiveness of the method of the present disclosure in terms of EM features, classification accuracy and comparison to state-of-the-art techniques.

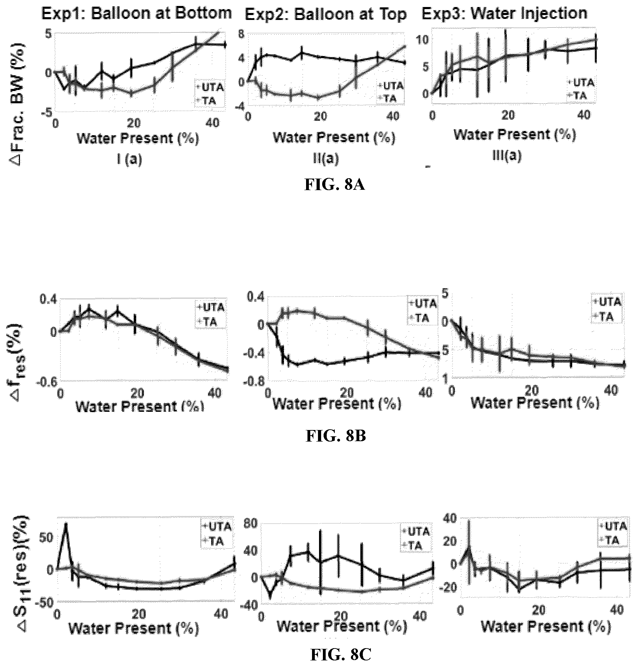

A. Computed EM Features: For computing EM features, $S_{11}$(dB) is measured over a frequency band of 3.7 to 4.3 GHz for all cases in the experiment design. Observed variations represent various oedema levels. From these measurements, features ($f_1$, $f_2$ and $f_3$) are extracted. FIGS. 8A through 8C depict exemplary graphical representations illustrating trends of each of the plurality of EM features for different experimental scenarios for untuned antennas and tuned antennas, according to an embodiment of the present disclosure. For all cases, it is observed that these parameters change significantly with change in water volume. For Exp-1: "Balloon at Bottom" and Exp-3: "Water Injection", the trends shown by the UTA and TA for features $f_1$ and $f_2$ are similar and a fixed offset is present between the two. However, for Exp-2, opposing trends are shown by the UTA and TA. This could be attributed to peculiarity in Exp-2. Distance between water top surface and the antenna remains constant for Exp-2 unlike Exp-1 and Exp-3. Further, the trends exhibited by $f_3$ for all three cases, do not follow a predetermined manner. It is also noted that the three features, in each of these experiments show distinguishable trends for the low ((5-20%) and high (>20%) water accumulation. In essence, while all the three features display distinguishable characteristics, the trends exhibited by them require complex analytical modeling for inference. Thus, oedema severity characterization merely from the $S_{11}$ data or these extracted features seem unfeasible. This necessitates the utilization of a ML-based approach to assess complex patterns of the three parameters together for oedema severity classification.

B. Classification Results: Each experiment (Exp-1, Exp-2 and Exp-3) has been repeated multiple times (4 trials/times). Three trials were chosen for training and validation using 5-fold cross validation to yield a trained classifier which was then tested on the remaining trial data. This was repeated for all possible combinations. Table 2 below provides mean $\pm$ standard deviation (*Std*) of classification accuracies *(CA)* for both UTA and TA for various feature combinations using the classifier.

Table 2

| Experimental Scenario | Feature | Classification Accuracy (CA) (Mean $\pm$ Std) | |
|---|---|---|---|
| | | Untuned Antenna | Tuned Antenna |
| **Exp-1: Balloon at Bottom** | $f_1$ | 63.9 $\pm$ 6.81 | 63.9$\pm$19.84 |
| | $f_2$ | 63.86$\pm$14.42 | 77.76$\pm$4.11 |
| | $f_3$ | 58.35$\pm$ 9.64 | 72.92$\pm$4.51 |
| | $\{f_1, f_2\}$ | 77.76$\pm$7.94 | 91.67$\pm$7.99 |
| | $\{f_1, f_3\}$ | 79.15$\pm$14.45 | 85.4$\pm$10.49 |
| | $\{f_2, f_3\}$ | 75.02$\pm$11.78 | 93.76$\pm$4.03 |
| | $\{f_1, f_2, f_3\}$ | 72.93$\pm$7.946 | **93.78$\pm$4.15** |
| **Exp-2: Balloon at Top** | $\{f_2, f_3\}$ | 50.03$\pm$14.38 | 85.35+19.22 |
| | $\{f_1, f_2, f_3\}$ | 69.46$\pm$4.79 | **85.42$\pm$18.50** |
| **Exp-2: Water Injected** | $\{f_2, f_3\}$ | 62.47$\pm$14.42 | 61.55$\pm$11.40 |
| | $\{f_1, f_2, f_3\}$ | 70.85$\pm$8.3 <br> 75$\pm$6.77 (Ensemble) | 70.47$\pm$7.55 <br> **77.25$\pm$7.09** (Ensemble) |

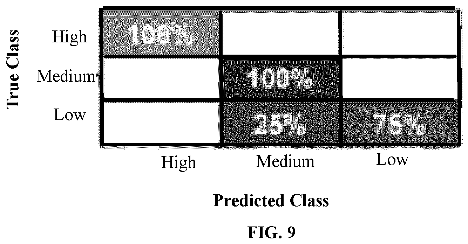

For all the experimental scenarios, combination of all three features performs better. It is observed that none of the features, only by themselves, are capable of classifying water accumulation extent/severity. Additionally, the classification accuracy shows that the TA antenna performs better for all the cases and feature combination, with highest accuracy of 93.78$\pm$4.15%. FIG. 9 illustrates a confusion matrix for a classifier used for unobtrusive oedema severity classification and quantification using microwave sensing, according to an embodiment of the present disclosure. The confusion matrix for the classifier using $\{f_1, f_2, f_3\}$ for Exp-1 shows that the error is higher in case of LOW oedema class. In this case, 25% of instances with water <5% are wrongly classified as MEDIUM category oedema. The classifier used in the present disclosure (e.g., $SVM_{Quad}$ ) has been compared to other classifiers like linear/quadratic discriminant analysis, k-nearest neighbour, Naïve Bayesian, Ensemble and neural network. In all the cases, the classifier $SVM_{Quad}$ appears to have outperformed. Only for Exp-3, $SVM_{Quad}$ exhibits almost similar accuracy for TA and UTA, where the ensemble method performed better. In comparison to existing methods, the present disclosure uses an unobtrusive, real-time and continuous monitoring technique for oedema severity detection. Unlike existing methods, the method of the present disclosure is not contact based, uses no harmful radiation, is low-cost, facile and rapid, along with requiring no preparation time.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method (200), comprising:

    acquiring (202), via one or more hardware processors, a plurality of scan measurements at a plurality of locations of a sample under test using a tuned antenna, wherein the tuned antenna is a microstrip patch antenna operating at a specific operating frequency with one or more optimized dimensions and positioned at a predefined distance from a numerical biological phantom;
    determining (204), via the one or more hardware processors, a plurality of reflection parameters from the plurality of scan measurements at each location from the plurality of locations of the sample under test using a vector network analyzer, wherein the vector network analyzer utilizes microwave sensing for determining the plurality of reflection parameters;
    extracting (206), via the one or more hardware processors, a plurality of physics-based electromagnetic (EM) features using a predetermined resonant frequency and a corresponding value of the plurality of reflection parameters at the predetermined resonant frequency to obtain a feature vector, wherein the feature vector comprises a training feature vector and a testing feature vector;
    classifying (208), via the one or more hardware processors, the sample under test into one of (i) a first oedema severity category, and (ii) a second oedema severity category, using a trained classifier model for the testing feature vector, wherein the trained classifier model is trained using the training feature vector; and
    quantifying (210), via the one or more hardware processors, the oedema based on a water percentage in the sample under test that is classified into the first oedema severity category and the second oedema severity category, using a trained regression model for a specific set of testing feature vector from the testing feature vector.

2. The processor implemented method as claimed in claim 1, wherein the sample under test is a biological tissue.

3. The processor implemented method as claimed in claim 1, wherein the specific operating frequency of the tuned antenna is 4 Giga Hertz (GHz) and the predefined distance of the tuned antenna from the numerical human phantom is 10 millimeter (mm).

4. The processor implemented method as claimed in claim 1, wherein the plurality of physics-based electromagnetic (EM) features specify water accumulation in the sample under test that represents a detected oedema.

5. The processor implemented method as claimed in claim 1, wherein the first oedema severity category represents a low oedema severity category, and the second oedema severity category represents a medium oedema severity category.

6. The processor implemented method as claimed in claim 1, wherein the step of classifying comprises classifying the sample under test into a third oedema severity category that represents a low oedema severity category.

7. A system (100), comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

acquire a plurality of scan measurements at a plurality of locations of a sample under test using a tuned antenna, wherein the tuned antenna is a microstrip patch antenna operating at a specific operating frequency with one or more optimized dimensions and positioned at a predefined distance from a numerical biological phantom;
determine a plurality of reflection parameters from the plurality of scan measurements at each location from the plurality of locations of the sample under test using a vector network analyzer, wherein the vector network analyzer utilizes microwave sensing for determining the plurality of reflection parameters;
extract a plurality of physics-based electromagnetic (EM) features using a predetermined resonant frequency and a corresponding value of the plurality of reflection parameter at the predetermined resonant frequency to obtain a feature vector, wherein the feature vector comprises a training feature vector and a testing feature vector;
classify the sample under test into one of (i) a first oedema severity category and (ii) a second oedema severity category, using a trained classifier model for the testing feature vector, wherein the trained classifier model is trained using the training feature vector; and
quantify the oedema based on a water percentage in the sample under test that is classified into the first oedema severity category and the second oedema severity category, using a trained regression model for a specific set of testing feature vector from the testing feature vector.

8. The system as claimed in claim 7, wherein the sample under test is a biological tissue.

9. The system as claimed in claim 7, wherein the specific operating frequency of the tuned antenna is 4 Giga Hertz (GHz) and the predefined distance of the tuned antenna from the numerical human phantom is 10 millimeter (mm).

10. The system as claimed in claim 7, wherein the plurality of physics-based electromagnetic (EM) features specify water accumulation in the sample under test that represents a detected oedema.

11. The system as claimed in claim 7, wherein the first oedema severity category represents a low oedema severity category, the second oedema severity category represents a medium oedema severity category.

12. The system as claimed in claim 7, wherein the step of classifying comprises classifying the sample under test into a third oedema severity category that represents a low oedema severity category.

13. One or more non-transitory machine-readable information storage mediums comprising one or more instructions which when executed by one or more hardware processors cause:

acquiring a plurality of scan measurements at a plurality of locations of a sample under test using a tuned antenna, wherein the tuned antenna is a microstrip patch antenna operating at a specific operating frequency with one or more optimized dimensions and positioned at a predefined distance from a numerical biological phantom; determining a plurality of reflection parameters from the plurality of scan measurements at each location from the plurality of locations of the sample under test using a vector network analyzer, wherein the vector network analyzer utilizes microwave sensing for determining the plurality of reflection parameters;

extracting a plurality of physics-based electromagnetic (EM) features using a predetermined resonant frequency and a corresponding value of the plurality of reflection parameters at the predetermined resonant frequency to obtain a feature vector, wherein the feature vector comprises a training feature vector and a testing feature vector; classifying the sample under test into one of (i) a first oedema severity category, and (ii) a second oedema severity category, using a trained classifier model for the testing feature vector, wherein the trained classifier model is trained using the training feature vector; and

quantifying the oedema based on a water percentage in the sample under test that is classified into the first oedema severity category and the second oedema severity category, using a trained regression model for a specific set of testing feature vector from the testing feature vector.

14. The one or more non-transitory machine-readable information storage mediums as claimed in claim 13, wherein the sample under test is a biological tissue.

15. The one or more non-transitory machine-readable information storage mediums as claimed in claim 13, wherein the specific operating frequency of the tuned antenna is 4 Giga Hertz (GHz) and the predefined distance of the tuned antenna from the numerical human phantom is 10 millimeter (mm).

16. The one or more non-transitory machine-readable information storage mediums as claimed in claim 13, wherein the plurality of physics-based electromagnetic (EM) features specify water accumulation in the sample under test that represents a detected oedema.

17. The one or more non-transitory machine-readable information storage mediums as claimed in claim 13, wherein the first oedema severity category represents a low oedema severity category, and the second oedema severity category represents a medium oedema severity category.

18. The one or more non-transitory machine-readable information storage mediums as claimed in claim 13, wherein the step of classifying comprises classifying the sample under test into a third oedema severity category that represents a low oedema severity category.

**FIG. 1**

FIG. 2

Acquiring, a plurality of scan measurements at a plurality of locations of a sample under test using a tuned antenna, wherein the tuned antenna is a microstrip patch antenna operating at a specific operating frequency with one or more optimized dimensions and positioned at a predefined distance from a numerical biological phantom ⟿ 202

Determining, a plurality of reflection parameters from the plurality of scan measurements at each location from the plurality of locations of the sample under test using a vector network analyzer, wherein the vector network analyzer utilizes microwave sensing for determining the plurality of reflection parameters ⟿ 204

Extracting, a plurality of physics-based electromagnetic (EM) features using a predetermined resonant frequency and a corresponding value of the plurality of reflection parameter at the predetermined resonant frequency to obtain a feature vector, wherein the feature vector comprises a training feature vector and a testing feature vector ⟿ 206

Classifying, via the one or more hardware processors, the sample under test into one of (i) a first oedema severity category and (ii) a second oedema severity category, using a trained classifier model for the testing feature vector, wherein the trained classifier model is trained using the training feature vector ⟿ 208

Quantifying, via the one or more hardware processors, the oedema based on a water percentage in the sample under test that is classified into the first oedema severity category and the second oedema severity category, using a trained regression model for a specific set of testing feature vector from the testing feature vector ⟿ 210

200 ⟿ **FIG. 3**

EP 4 740 843 A1

FIG. 4A

FIG. 4B

FIG. 5A

16

FIG. 5B

FIG. 6A

FIG. 6B

FIG. 6C

FIG. 6D

**FIG. 7A**

**Balloon at Top**

~3cm

1.5 cm

5.5 cm

7 cm

FIG. 7B

**1.5 cm**

~3cm

**Water Injection**

**5.5 cm**

**7 cm**

FIG. 7C

**FIG. 8A**

**FIG. 8B**

**FIG. 8C**

Predicted Class

FIG. 9

| | Europäisches Patentamt European Patent Office Office européen des brevets | **EUROPEAN SEARCH REPORT** | **Application Number** EP 25 20 3543 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GANGULY PRAPTI ET AL: "Predictive Modeling of Lung Water Content with Machine Learning and RF Sensor", 2024 IEEE CONFERENCE ON ANTENNA MEASUREMENTS AND APPLICATIONS (CAMA), IEEE, 9 October 2024 (2024-10-09), pages 1-5, XP034906540, DOI: 10.1109/CAMA62287.2024.10986198 [retrieved on 2025-05-09] * the whole document * * page 1 - page 5; figures 1, 2, 7, 8 * | 1-18 | INV. A61B5/00 A61B5/0507 |
| A | ZHANG HAOLIN ET AL: "A Preliminary Experiment Based on One-step Measurement-trained Supervised Descent Method for Microwave Thorax Imaging", 2021 PHOTONICS & ELECTROMAGNETICS RESEARCH SYMPOSIUM (PIERS), IEEE, 21 November 2021 (2021-11-21), pages 1921-1930, XP034078688, DOI: 10.1109/PIERS53385.2021.9695085 [retrieved on 2022-01-26] * the whole document * | 1-18 | |
| A | GAGARIN RUTHSENNE ET AL: "Determination of pulmonary edema using microwave sensor array: Simulation studies with anatomically realistic human CAD-models", 2013 IEEE ANTENNAS AND PROPAGATION SOCIETY INTERNATIONAL SYMPOSIUM (APSURSI), IEEE, 7 July 2013 (2013-07-07), pages 2189-2190, XP032555908, ISSN: 1522-3965, DOI: 10.1109/APS.2013.6711753 ISBN: 978-1-4799-3538-3 [retrieved on 2014-01-13] * the whole document * | 1-18 | |

TECHNICAL FIELDS SEARCHED (IPC)

A61B

The present search report has been drawn up for all claims

| Place of search | Date of completion of the search | Examiner |
|---|---|---|
| The Hague | 14 January 2026 | Knoop, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421085558